# EUROPEAN PATENT APPLICATION

(11) **EP 2 478 890 A1**
(43) Date of publication of application: **25.07.2012**
(21) Application number: 12164702.8
(22) Date of filing: 09.02.2009
(51) Int. Cl.: A61K 8/04, A61K 8/81, A61K 8/34, A61K 8/35, A61K 8/37, A61K 8/40, A61Q 17/04

(54) **Photoprotective compositions with caprylyl glycol and styrene/acrylate copolymer**

(30) Priority: 11.02.2008 US 27672
(62) Divisional of application: 09710289.1
(71) Applicant: Playtex Products, Llc, Shelton, CT 06484 (US)
(72) Inventor: Spaulding, Laura A., Wayne, NJ New Jersey 07470 (US)
(74) Representative: Neelissen-Subnel, Marianne

(57) **Abstract**

A photoprotective composition includes one or more photoactive agents (such as a UV filter compound), one or more optimizing agents, and a copolymer booster. Such a composition provides an unexpected synergistic combination of the one or more optimizing agents and the copolymer booster for increasing at least one of SPF, UVA/UVB ratio, critical wavelength, photostability of UVA absorber and PFA, and any combinations thereof, of the composition.

## Description

### CROSS-REFERENCE TO RELATED APPLICATIONS

This patent application claims priority benefit under 35 U.S.C. §119(e) of copending, U.S. Provisional Patent Application, Ser. No. 61/027,672, filed February 11, 2008, the disclosure of which is incorporated by reference herein in its entirety.

### BACKGROUND OF THE INVENTION

### 1. Field of the Invention

This application relates to photoprotective compositions and in particular to topical photoprotective compositions for human use.

### 2. Related Art

Sunscreen compositions are applied to the skin to protect the skin from the sun's ultraviolet rays that can lead to erythema (sunburn).

Sunlight or ultraviolet radiation in the UV-B range has a wavelength of 290 nanometers (nm) to 320 nm and is known to be the primary cause of sunburn. Ultraviolet rays at a wavelength of 320 nm to 400 nm, known as UV-A radiation, produce tanning of the skin. However, in the process of doing so, the UV-A rays can damage or harm the skin.

Besides the immediate malady of sunburn, excessive sunlight exposure can lead to other skin disorders. For instance, prolonged exposure to the sun may lead to actinic keratoses and carcinomas. Another long-term effect of sun exposure is premature aging of the skin. This condition is characterized by skin that is wrinkled, cracked and has lost its elasticity.

As stated above, sunscreens are typically formulated with the goal of inhibiting skin damage from the sun's rays. The sunscreen composition filters or blocks the harmful UV-A and UV-B rays that can damage and harm the skin. It is believed that sunscreen agents (sometimes referred to herein as "photoactive agents") accomplish this by absorbing the UV-A and/or UV-B rays.

Typically, UV-B filters are combined with the UV-A filters in a solution with other lipophilic or oily ingredients (collectively referred to as "sunscreen actives") and solvents to form an oil phase. The solvents are used to dissolve the sunscreen actives into the oil phase. Typically, but not necessarily, the oil phase is dispersed with the help of emulsifiers and stabilizers into an aqueous solution composed primarily of water, to make an emulsion, which becomes the final sunscreen composition.

One problem associated with the use of UV filters is that they are not photostable and will degrade rapidly and exponentially when exposed to UV radiation. The organic UV-A filters most commonly used in commercial sunscreen compositions are the dibenzoylmethane derivatives, particularly 4-(1, 1-dimethylethyl)-4'-methoxydibenzoylmethane (also called avobenzone and sold under the brand name PARSOL 1789).

It is also well known that the above described UV-A filters, particularly the dibenzoylmethane derivatives, can suffer rapid photochemical degradation when used alone or when combined with the most commercially used UV-B filters described above. Thus, the efficiency of the sunscreen composition (i.e., SPF, PFA, critical wavelength, Star Rating) containing these photoactive agents is compromised, unless the photodegradation is controlled by improving the photostability of the system in UVA and/or UVB regions.

The absorption spectrum of a sunscreen is characterized by the critical wavelength, which is the wavelength where the integral of the spectral absorbance curve reaches 90% of the integral from 290 nm to 400 nm. In other words, when the absorbance exhibited by a sunscreen product at wavelengths from 290 nm to 400 nm is plotted in a curve on a graph, 90% of the area under the curve is between the critical wavelength and 290 nm. The critical wavelength indicates the breadth of UV protection and is often employed to evaluate the long wave efficacy of sunscreen products. Therefore, by optimizing the critical wavelength properties of a photoprotective composition, enhanced photoprotection may result.

Another measure of a sunscreen composition's efficacy is the Star Rating (UVA/UVB Ratio) according to the Boots Star Rating System (once a 4-star system that was recently revised to include a 5-star category). The Star Rating is calculated as an indicator of the UVA absorbance properties of a sunscreen product, relative to UVB as described in the Revised Guidelines to the practical measurement of UVA:UVB ratios according to the Boots Star Rating System. The calculation of the UVA:UVB absorbance ratio will typically yield values from zero (equal to no UVA absorbance) up to 1.0 (UVA absorbance equal to UVB).

### SUMMARY OF THE INVENTION

The present invention resides in one aspect in a photoprotective composition that includes one or more photoactive agents, one or more optimizing agents, and a copolymer booster. In one embodiment, an optimizing agent includes caprylyl glycol, and the copolymer booster includes styrene/acrylate copolymer spheres. As demonstrated herein, such a composition provides a synergistic combination of the one or more optimizing agents and the copolymer booster for increasing at least one of SPF, UVA/UVB ratio, critical wavelength, photostability of UVA absorber and PFA, and any combinations thereof. In one embodiment, where the optimizing agent includes caprylyl glycol and the copolymer booster includes styrene/acrylate copolymer spheres, the photoprotective composition achieves surprising and unexpectedly good SPF performance.

In various embodiments of the invention, the caprylyl glycol may comprise about 0.1% to about 10% of the composition by weight, and the styrene/acrylate copolymer spheres may comprise about 0.1% to about 10% of the composition by weight.

According to another aspect, the present invention is directed to a photoprotective composition in the form of an emulsion that comprises, by weight of the composition, a photoactive agent in an amount of about 0.1% to about 40% and an optimizing agent in an amount of about 0.1% to about 40%, the optimizing agent comprising caprylyl glycol. The photoprotective composition also includes styrene/acrylate copolymer spheres in an amount of about 0.1 to about 10%, water in an amount of about 40% to about 90%, and an emulsifier in an amount of about 0.01% to about 10%. The pH of the composition may be about 3 to about 9.

The composition may also include a thickening agent, emollient, SPF booster, moisturizer, humectant, film former/waterproofing agent, bio-active (functional) ingredient, pH adjuster/chelating agent, preservative, fragrance, effect pigment, color additive, lubricant, elastomer, or any combination thereof;

The photoprotective compositions of the present invention may also include caprylyl glycol in an amount about 0.25% to about 5%, or about 0.5% to about 2.5%.

In another aspect, the photoprotective composition may include the styrene/acrylate copolymer spheres in an amount of about 3% to about 7%, optionally in an amount of about 4% to about 6%.

The composition may also comprise an emulsion and may include water, one or more emulsifiers, thickening agents, emollients, SPF boosters, moisturizers, humectants, film former/waterproofing agents, bio-active (functional) ingredients, pH adjusters/chelating agents, preservatives, fragrances, effect pigments, color additives, lubricants, elastomers, or any combination thereof.

### DETAILED DESCRIPTION OF PREFERRED EMBODIMENTS

The present invention is directed to photoprotective compositions that comprise one or more photoactive agents, one or more optimizing agents such as a glycol, and a copolymer booster such as styrene/acrylate copolymer spheres. As described herein, the photoprotective compositions exhibit unexpected synergistic performance in connection with one or more of the following properties: boosting the SPF, UVA/UVB ratio (Star Rating), critical wavelength, photostability of UVA absorber and PFA, and any combinations thereof. The unexpected synergistic performance results in a more efficient UV radiation-absorbing composition. The styrene/acrylate copolymer spheres in a photoprotective composition boost SPF values across the UV region (UVA and UVB) and work equally well with organic and inorganic sunscreen actives. As illustrated by the examples herein, photoprotective compositions according to this invention may comprise emulsions, including oil-in-water emulsions and water-in-oil emulsions. In a preferred embodiment, the styrene/acrylate copolymer spheres are added into the oil phase. In another embodiment, the styrene/acrylate copolymer spheres are added into the water phase. The present invention is described herein by reference to a sunscreen composition for use on mammalian hair and/or skin, but the principles set forth herein apply equally to photoprotective compositions used for other purposes as well.

One commercially available styrene/acrylate copolymer sphere material suitable for the present invention is SunSpheres™ polymer spheres sold by Rohm and Haas Company. SunSpheres™ polymer spheres are hollow styrene/acrylate copolymer spheres that are manufactured via emulsion polymerization. SunSpheres™ polymer spheres have a mean particle size of about 100 micrometers. Solid SunSpheres™ polymer spheres take the form of agglomerates that must be broken down to liberate the individual spheres and yield the desired effect in the photoprotective composition. The solid SunSpheres™ agglomerates may be broken down in various ways, for example, by a high-shear process such as homogenization of an aqueous dispersion of the spheres before forming the emulsion.

For human topical use, a photoprotective composition according to this invention may comprise about 0.1% to about 10 % styrene/acrylate copolymer spheres, by weight, based on the weight of the composition. Unless otherwise specified, all percentages stated herein are weight percents based on a finished photoprotective composition, i.e., w/w%. In various embodiments, a composition as described herein may comprise about 3 to about 7%; about 4% to about 6%; about 0.25% to about 5%, or about 0.5% to about 1.25% styrene/acrylate copolymer spheres. These photoprotective compositions can achieve an in-vivo SPF up to about 85. However, the present invention is not limited in this regard as other embodiments with lower SPF ratings are also encompassed by the present invention. In all embodiments, the synergistic performance of the styrene/acrylate copolymer spheres and glycol optimizing agent allows for the use of lesser amounts of the sunscreen actives than would otherwise be needed to achieve the desired SPF performance.

As illustrated in the examples below, one glycol that may be included in a photoprotective composition with styrene/acrylate copolymer spheres is caprylyl glycol (1,2-octanediol), which functions as an optimizing agent and has good antimicrobial activity. For human topical use, a photoprotective composition as described herein may comprise caprylyl glycol in an amount corresponding to about 0.1% to about 10% (e.g., to 11%), optionally about 0.25% to about 5% or about 0.5% to about 1.25%, by weight of the photoprotective composition.

Photoactive agents are compounds that respond to UV radiation photoelectrically. Examples of photoactive agents include, but are not limited to, UV filters, pigments, or dyes. Photoactive agents suitable for use in the sunscreen composition of the present invention include one or more UV filters. Suitable UV filters may include, but are not limited to, one or more compounds selected from the following categories (with specific examples): Tinasorb, e.g. Tinasorb MR (methylene bisbenzotriazol tetramethylbutylphenol), and Tinasorb SR (anizotriazine); Ecamsule (e.g., trade name Mexoryl® SX; Terephthalylidene Dicamphor Sulfonic Acid); p-aminobenzoic acid, its salts and its derivatives (ethyl, isobutyl, glyceryl esters; p-dimethylaminobenzoic acid); anthranilates (o-aminobenzoates; methyl, menthyl, phenyl, benzyl, phenylethyl, linalyl, terpinyl, and cyclohexenyl esters); salicylates (octyl, amyl, phenyl, benzyl, menthyl (homosalate), glyceryl, and dipropyleneglycol esters); cinnamic acid derivatives (menthyl and benzyl esters, alpha-phenyl cinnamonitrile; butyl cinnamoyl pyruvate); dihydroxycinnamic acid derivatives (umbelliferone, methylumbelliferone, methylaceto-umbelliferone); camphor derivatives (3-benzylidene, 4-methylbenzylidene, polyacrylamidomethyl benzylidene, benzalkonium methosulfate, benzylidene camphor sulfonic acid, and terephthalylidene dicamphor sulfonic acid); trihydroxycinnamic acid derivatives (esculetin, methylesculetin, daphnetin, and the glucosides, esculin and daphnin); hydrocarbons (diphenylbutadiene, stilbene); dibenzalacetone and benzalacetophenone; naptholsulfonates (sodium salts of 2-naphthol-3,6-disulfonic and of 2-naphthol-6,8-disulfonic acids); dihydroxy-naphthoic acid and its salts; o- and p-hydroxydiphenyldisulfonates; coumarin derivatives (7-hydroxy, 7-methyl, 3-phenyl); diazoles (2-acetyl-3-bromoindazole, phenyl benzoxazole, methyl naphthoxazole, various aryl benzothiazoles); quinine salts (bisulfate, sulfate, chloride, oleate, and tannate); quinoline derivatives (8-hydroxyquinoline salts, 2-phenylquinoline); hydroxy- or methoxy-substituted benzophenones; uric and vilouric acids; tannic acid and its derivatives; hydroquinone; benzophenones (oxybenzone, sulisobenzone, dioxybenzone, benzoresorcinol, 2,2',4,4'-tetrahydroxybenzophenone, 2,2'-dihydroxy-4,4'-dimethoxybenzophenone, octabenzone), dibenzoylmethane derivatives, avobenzone, 4-isopropyldibenzoylmethane, butylmethoxydibenzoylmethane, 4-isopropyl-dibenzoylmethane, octocrylene, drometrizole trisiloxane, and metal oxides (titanium dioxide, zinc oxide).

In one embodiment of the invention a photoactive agent is selected from the group consisting of UV-A filters, UV-B filters, or any combinations thereof. In a cosmetically-acceptable sunscreen embodiment for use on human skin, a photoactive agent preferably is selected from approved (if regulated), cosmetically-acceptable UV-A filters, UV-B filters, or any combinations thereof.

For example, for a product marketed in the United States, preferred cosmetically-acceptable photoactive agents and concentrations (by way of example, reported as a percentage by weight of the total cosmetic sunscreen composition) include: aminobenzoic acid (also called para-aminobenzoic acid and PABA; 15% or less), avobenzone (also called butyl methoxy dibenzoylmethane; 3% or less), cinoxate (also called 2-ethoxyethyl p-methoxycinnamate; 3% or less), dioxybenzone (also called benzophenone-8; 3% or less), homosalate (15% or less), menthyl anthranilate (also called menthyl 2-aminobenzoate; 5% or less), octocrylene (also called 2-ethylhexyl-2-cyano-3,3 diphenylacrylate; 10% or less), octyl methoxycinnamate (7.5% or less), octyl salicylate (also called 2-ethylhexyl salicylate; 5% or less), oxybenzone (also called benzophenone-3; 6% or less), padimate O (also called octyl dimethyl PABA; 8% or less), phenylbenzimidazole sulfonic acid (water soluble; 4% or less), sulisobenzone (also called benzophenone-4; 10% or less), titanium dioxide (25% or less), trolamine salicylate (also called triethanolamine salicylate; 12% or less), and zinc oxide (25% or less).

Other preferred cosmetically-acceptable photoactive agents and concentrations (by way of example, percent by weight of the total cosmetic sunscreen composition) include diethanolamine methoxycinnamate (10% or less), ethyl-[bis(hydroxypropyl)]aminobenzoate (5% or less), glyceryl aminobenzoate (3% or less), 4-isopropyl dibenzoylmethane (5% or less), 4-methylbenzylidene camphor (6% or less), terephthalylidene dicamphor sulfonic acid (10% or less), and sulisobenzone (also called benzophenone-4, 10% or less).

For a product marketed in the European Union, preferred cosmetically-acceptable photoactive agents and concentrations (by way of example, reported as a percentage by weight of the total cosmetic sunscreen composition) include: PABA (5% or less), camphor benzalkonium methosulfate (6% or less), homosalate (10% or less), benzophenone-3 (10% or less), phenylbenzimidazole sulfonic acid (8% or less, expressed as acid), terephthalidene dicamphor sulfonic acid (10% or less, expressed as acid), butyl methoxydibenzoylmethane (5% or less), benzylidene camphor sulfonic acid (6% or less, expressed as acid), octocrylene (10% or less, expressed as acid), polyacrylamidomethyl benzylidene camphor (6% or less), ethylhexyl methoxycinnamate (10% or less), PEG-25 PABA (10% or less), isoamyl p-methoxycinnamate (10% or less), ethylhexyl triazone (5% or less), drometrizole trielloxane (15% or less), diethylhexyl butamido triazone (10% or less), 4-methylbenzylidene camphor (4% or less), 3-benzylidene camphor (2% or less), ethylhexyl salicylate (5% or less), ethylhexyl dimethyl PABA (8% or less), benzophenone-4 (5%, expressed as acid), methylene bis-benztriazolyl tetramethylbutylphenol (10% or less), disodium phenyl dibenzimidazole tetrasulfonate (10% or less, expressed as acid), bis-ethylhexyloxyphenol methoxyphenol triazine (10% or less), methylene bisbenzotriazolyl tetramethylbutylphenol (10% or less, also called TINOSORB™ M), and bisethylhexyloxyphenol methoxyphenyl triazine (10% or less, also called TINOSORB S), Mexoryl® XL (also called drometrizole trisiloxane, 15% or less), Mexoryl® SX (15% or less).

One or more photoactive agents are present in the composition in an amount about 1% to about 40% by weight of the total weight of the sunscreen composition. The amount of photoactive agent in the composition can vary in the above range depending on the sun protection factor (SPF) desired. Usually, the higher the SPF, the greater the total amount of photoactive agent used in the composition. However, as demonstrated herein, the present invention provides formulating a photoprotective composition with an increased or boosted SPF without the inclusion of additional photoactive agent or increasing the total amount of photoactive agent in the composition.

Preferably, the one or more photoactive agents are included at about 2% to about 35% to achieve a SPF of about 2 to about 50. More preferably, the one or more photoactive agents are included in an amount about 4% to about 30% to achieve a SPF value of about 4 to about 45.

The compositions of the present invention include one or more optimization agents. It has been unexpectedly found that the inclusion of one or more optimization agents according to the present invention in a sunscreen composition results in a stable, efficient composition.

The one or more optimization agents may be present in a photoprotective composition according to the present invention from about 0.1% to about 40%, based on the total weight of the composition. Preferably, the one or more optimization agents are present from about 0.5% to about 15%, and more preferably from about 1% to about 10%, based on the total weight of the composition.

Suitable optimization agents for use in the present invention may include, but are not limited to, diols, alcohols, glycols, polyhydric alcohols, polyhydric alcohol derivatives having one or more hydroxyl groups, or any combinations thereof. Preferably, the one or more optimization agents are one or more alcohols. More preferably, the one or more optimization agents are one or more diols, glycols, or any combinations thereof. Most preferably, diols are 1,2-diols.

Suitable glycols for use in the invention include, but are not limited to, pentylene glycol (1,2-pentanediol), neopentyl glycol (neopentanediol), caprylyl glycol (1,2-octanediol), etoxydiglycol, butylene glycol monopropionate, diethylene glycol monobutyl ether, PEG-7 methyl ether, octacosanyl glycol, arachidyl glycol, benzyl glycol, cetyl glycol (1,2-hexanediol), C₁₄₋₁₈ glycol, C₁₅₋₁₈ glycol, lauryl glycol (1,2-dodecanediol), butoxydiglycol, 1,10-decanediol, ethyl hexanediol, or any combinations thereof.

The one or more optimization agents are preferably included in a solvent system used to dissolve the one or more photoactive agents. As used herein, a solvent system includes all of the compounds used to dissolve the one or more photoactive agents. By way of example, the solvent system for cosmetic sunscreen compositions will include one or more optimization agents that are lipophilic compounds for dissolving oil miscible photoactive agents and hydrophilic compounds for dissolving water miscible photoactive agents. In a preferred embodiment, the solvent system includes one or more optimization agents that are both oil miscible and water miscible. Such agents offer a distinct advantage when formulating the sunscreen composition, as the need for separate oil and water miscible solvents is reduced or eliminated.

### Additional Components

In addition to the one or more optimization agents, the solvent system may include, but is not limited to, one or more solvents, such as C₁₂₋₁₅ alkyl benzoates (e.g., Finsolv TN C₁₂₋₁₅), capric triglycerides, caprylic triglycerides, diethylhexyl adipate, diethylhexyl malate, diethylhexyl 2,6-naphthalate, ethylhexyl palmitate, ethylhexyl stearate, isoeicosane, isopropyl myristate, isopropyl palmitate, mineral oil, octyldodecyl neopentanoate, polyisobutene, PPG-2 myristyl ether propionate, cocoglycerides, isostearyl linoleate, diisopropyl adipate, myristyl ether myristate, octyl palmitate, propylene glycol ricinoleate, cetyl esters, propylene glycol laurate, or any combinations thereof.

In addition to the one or more photoactive agents and the one or more optimizing agents, any well-known cosmetically-acceptable additives, such as, for example, water, emulsifier, thickening agent, emollient, SPF booster, moisturizer, humectant, film former/waterproofing agent, bio-active (functional) ingredient, pH adjuster/chelating agent, preservative, fragrance, effect pigment, color additive, lubricant, elastomer, or any combinations thereof, and/or other common cosmetic formulation additives for solubility of sunscreen active compounds, emulsification, thickening and to provide other skin enhancement, e.g., moisturizing properties, may be included in the sunscreen composition.

The compositions of the present invention preferably include water. The water is present in the compositions of the present invention in an amount about 40% to about 90%, and preferably about 50% to about 80%, of the total weight of the compositions.

The compositions of the present invention may include one or more emulsifiers. The one or more emulsifiers suitable for use in the present invention include, but are not limited to, acrylates crosspolymer, acrylates/C₁₀₋₃₀ alkylacrylate crosspolymer, acrylates/vinyl isodecanoate crosspolymer, polyacrylic acid, sodium polymethacrylate, sodium polyacrylate, polyacrylates, cetyl alcohol, cetearyl alcohol, oleth-10, diethylhexyl esters, sorbitan oleate, sorbitan sesquioleate, sorbitan isostearate, sorbitan trioleate, PEG-20 almond glycerides, polyglyceryl-3-diisostearate, polyglycerol esters of oleic/isostearic acid, polyglyceryl-6 hexaricinolate, polyglyceryl-4-oleate, polygylceryl-4 oleate/PEG-8 propylene glycol cocoate, sodium glyceryl oleate phosphate, hydrogenated vegetable glycerides phosphate, or any combinations thereof.

The amount of emulsifier present in the compositions of the present invention is about 0.01% to about 10% of the total weight of the composition. Preferably, the emulsifier is present in an amount about 0.1% to about 5% of the total weight of the composition.

In an alternative embodiment, the sunscreen composition of the present invention may be formulated to be emulsifier free, which can improve water resistance of the sunscreen formulation and reduce its irritation potential.

One or more thickening agents that may be used in the compositions of the present invention. Suitable thickening agent includes, but is not limited to, one or more stabilizers, synthetic and natural gum or polymer products, polysaccharide thickening agents, associative thickeners, anionic associative rheology modifiers, nonionic associative rheology modifiers, oil-thickening agents, acrylates/C₁₀₋₃₀ alkyl acrylate crosspolymer, acrylates/aminoacrylates/_{C10-30} alkyl PEG-20 itaconate copolymer, acrylates copolymer, acrylates/steareth-20 methacrylate copolymer, acrylates/beheneth-25 methacrylate copolymer, PEG-150/decyl alcohol/SMDI copolymer, PVP, PVM/MA decadiene crosspolymer, carbomer, PEG crosspolymer, acrylates/palmeth-25 acrylates copolymer, polysaccharides, polyacrylates, polyether-1, sodium magnesium silicate, sodium carbomer, sodium polyacrylate, sodium polymethacrylate, sodium polyacryloyldimethyl taurate, sodium acryloyldimethyl taurate copolymer, sodium carragenan, sodium carboxymethyl dextran, hydroxyethylcellulose, hydroxypropyl cyclodextran, bentonites, trihydroxystearin, aluminum-magnesium hydroxide stearate, xanthan gum, or any combinations thereof.

The amount of thickening agent present in the compositions of the present invention is about 0.01% to about 10% of the total weight of the composition. Preferably, the thickener is present in an amount about 0.1% to about 5% of the total weight of the composition.

The present compositions may include one or more emollients. An emollient provides a softening, protective or soothing effect on the skin surface and is generally considered safe for topical use. It also helps control the rate of evaporation and the tackiness of the compositions.

Suitable emollients include, for example, cocoglycerides, cyclomethicone, dimethicone, dicapryl maleate, caprylic/capric triglyceride, isopropyl myristate, octyl stearate, isostearyl linoleate, lanolin oil, coconut oil, cocoa butter, olive oil, avocado oil, aloe extracts, jojoba oil, castor oil, fatty acid such as oleic and stearic, fatty alcohol such as cetyl and hexadecyl, diisopropyl adipate, hydroxybenzoate esters, benzoic acid esters of C₉₋₁₅ alcohols, isononyl iso-nonanoate, alkanes such as mineral oil, silicone such as dimethyl polysiloxane, ether such as polyoxypropylene butyl ether and polyoxypropylene cetyl ether, C₁₂₋₁₅ alkyl benzoate, or any combinations thereof.

The total amount of emollient present in the compositions is typically about 0.1% to about 30% of the total weight of the composition. Preferably, emollient is present in an amount about 1% to about 20% of the total weight of the composition.

The pH of the compositions of the present invention may be adjusted by one or more basic pH adjusters and/or chelating agents. For example, sodium hydroxide, triethanolamine (TEA), EDTA salts, or any combinations thereof are suitable pH adjusters/chelating agents that may be included in the sunscreen compositions of the present invention.

An effective amount of a pH adjuster and/or chelating agent is included to adjust the pH of the final compositions to about 3 to about 9. Preferably, the pH is adjusted to about 5 to about 8 and more preferably about 6 to about 7.

One or more humectants may be used in the compositions of the present invention. Suitable humectants include, but are not limited to, glycerin, pentylene glycol, hexylene glycol, propylene glycol, butylene glycol, sorbitol, PEG-4, or any combinations thereof.

One or more humectants may be included in the compositions of the present invention in an amount about 0.1% to about 15% of the total weight of the composition. Preferably, humectant is present in an amount about 1% to about 5% of the total weight of the composition.

The present compositions may include one or more SPF boosters. SPF booster itself is not an active ingredient, but is designed to enhance the effectiveness of the photoactive agents actives present in the formulation. Suitable SPF boosters include, but are not limited to, styrene/acrylates copolymer, sodium bentonites, highly purified white sodium bentonites, montmorillonite, hydrogels, fluorene derivatives, ester derivatives of cyano(9H-fluoren-9-ylidene), amides, malates, bis-urethanes, or any combinations thereof. A preferred styrene/acrylates copolymer for use in the present invention is sold under the trade name SunSpheres® by Rohm and Haas Company.

When present, the one or more SPF boosters may be included in the compositions of the present invention in an amount about 0.1% to about 10% of the total weight of the composition, optionally about 1% to about 6% by weight. In a specific embodiment, an SPF booster is present in an amount about 3% to about 5% of the total weight of the composition.

Another component that may be used in the compositions of the present invention is a film former/waterproofing agent. The film former/waterproofing agent is a hydrophobic material that imparts film forming and waterproofing characteristics to the emulsion. Suitable film former/waterproofing agent for use in the compositions of the present invention include, but is not limited to, acrylates/acrylamide copolymer, acrylates copolymer, acrylates/C₁₂₋₂₂ alkylmethacrylate copolymer, polyethylene, waxes, VP/Dimethiconylacrylate/Polycarbamyl polyglycol ester, butylated PVP, PVP/Hexadecene copolymer, octadecene/MA copolymer, PVP/Eicosene copolymer, tricontanyl PVP, Brassica Campestris/Aleuritis Fordi Oil copolymer, decamethyl cyclopentasiloxane (and) trimethylsiloxysilicate, or any combinations thereof.

One or more film formers/waterproofing agents may be present in the compositions of the present invention in an amount about 0.1% to about 5% of the total weight of the composition. Preferably, the one or more film formers/waterproofing agents is present in the compositions of the present invention in an amount about 1% to about 3% of the total weight of the composition.

In addition, it has been unexpectedly found that the acrylates/C₁₂₋₂₂ alkylmethacrylate copolymer may protect the lipids in a user's skin by imparting structure to the epidermal lipids in the skin (stratum corneum) and sebaceous lipids (sebum) and preventing them from depletion. As a result, it is believed that the acrylates/C₁₂₋₂₂ alkylmethacrylate copolymer may enhance and help to maintain the barrier properties of the lipid barrier in stratum corneum.

Therefore, the compositions of the present invention may exhibit moisturizing and anti-inflammatory properties without a need for including moisturizers and/or anti-inflammatory agents in the compositions.

One or more preservatives may be included in the compositions of the present invention. The preservative protects the compositions from microbial contamination and/or oxidation. As such, the preservative can include an antioxidant. Preservatives, such as diazolidinyl urea, iodopropynyl butylcarbamate, chloromethylisotiazolinone, methylisothiazolinone, vitamin E and its derivatives including vitamin E acetate, vitamin C, butylated hydroxytoluene, butylparaben, ethylparaben, methylparaben, propylparaben, isobutylparaben, phenoxyethanol, or any mixtures thereof, may be included as a preservative in a composition of the present invention.

About 0.01% to about 2% of preservative may be included in a composition of the present invention. Preferably, one or more preservatives total about 0.5% to about 1.5% of the total weight of the composition.

The compositions of the present invention may also have other optional additives including bio-active (functional) ingredients. For instance, one or more plant extracts, fruit extracts, vegetable extracts, algae extracts, sugars, polysaccharides, lipids, proteins, peptides, aminoacids, aminoacid derivatives, absorbents, elastomers, for example DC 9011 silicone elastomer blend (Dow Corning) (cyclopentasiloxane (and) PEG-12 (and) dimethicone crosspolymer), salicylic acid, alpha and beta hydroxy acids, oil and water soluble vitamins including vitamins A, C, and E and their derivatives, or any mixtures thereof, may be included in the sunscreen compositions.

When present, the optional additives may be included in the present composition in an amount about 0.001% to about 10%, based on the total weight of the composition.

The photoprotective compositions disclosed herein can be produced in a variety of forms, including paste, lotions, creams, ointments, gels, solid sticks, emulsions, aerosols, solutions, dispersions, liquid form for pump spray, or any other forms of cosmetic compositions, as well as for paints, coatings, and the like.

Illustrative formulations for sample water-in-oil emulsion photoprotective compositions according to this invention (formulations A, H, I, J, and K) and some comparative formulations (formulations B, C, D, E, F and G) are indicated in the following Table 1. Ingredients are shown with percent by weight of the sample formulation. In formulations B, C, D and E, the Finsolve TN was included to replace the weight percent that the styrene/acrylate copolymer spheres and /or caprylyl glycol that would be present in a photoprotective composition according to this invention, to keep the emulsifier system and water amount constant and minimize the effect on the emulsion structure. The styrene/acrylate copolymer spheres in the formulations of Table 1 were Rohm & Haas Sunspheres®. In Table 1, ingredients in each formulation are stated in weight percent by weight of the composition including all the ingredients therein.

An optional method of preparing the sunscreen compositions is to add the carbopol to the water in a container, allow the carbopol to hydrate and then heat the carbopol and water to about 40°C; slurry the xanthan gum into the glycerin and add the slurry to the container with continued heat; raise the temperature to about 60 to 65°C, add the sarkosyl and the Tween 60 polysorbate to produce a phase "A"; heat the phase A to about 72 to 76°C, separately combine the other ingredients (except for TEA, fragrance, aloe barbadensis and vitamin E) to provide a phase "B", heat the phase B to about 72 to 76°C; start homogenizing phase A while slowly adding phase B; and add the TEA with mixing, cool to about 38 - 40°C and add the remaining ingredients.

**Table 1**

| | Formulation | | | | | | | | | | |
|---|---|---|---|---|---|---|---|---|---|---|---|
| Ingredients (described below as needed) | A | B | C | D | E | F | G | H | I | J | K |
| Water | q.s | | | | | | | | | | |
| Gellant | 0.5 to 2.0 | | | | | | | | | | |
| Emulsifier | 0.5 to 5.0 | | | | | | | | | | |
| Chelant | 0 to 0.2 | | | | | | | | | | |
| Film former | 0.5 to 2.0 | | | | | | | | | | |
| Ester emollient | 0.5 to 7.0 | | | | | | | | | | |
| Viscosity increasing agent | 0 to 1.0 | | | | | | | | | | |
| Preservative | 0.2 to 1.5 | | | | | | | | | | |
| Fragrance | 0 to 0.2 | | | | | | | | | | |
| Skin conditioning agent | 0 to 0.1 | | | | | | | | | | |
| pH adjuster | 0 to 1.0 | | | | | | | | | | |
| Benzophenone-3 | 6.0 | 6.0 | 6.0 | 6.0 | 0 | 6.0 | 6.0 | 6.0 | 6.0 | 6.0 | 6.0 |
| Avobenzone | 3.0 | 3.0 | 3.0 | 3.0 | 0 | 3.0 | 3.0 | 3.0 | 3.0 | 2.0 | 2.0 |
| Octocrylene | 2.4 | 2.4 | 2.4 | 2.4 | 0 | 2.4 | 2.4 | 2.4 | 2.4 | 1.6 | 1.6 |
| Homosalate | 15.0 | 15.0 | 15.0 | 15.0 | 0 | 15.0 | 15.0 | 15.0 | 15.0 | 10.0 | 15.0 |
| Octisalate (aka octyl salicylate) | 5.0 | 5.0 | 5.0 | 5.0 | 0 | 5.0 | 5.0 | 5.0 | 5.0 | 5.0 | 5.0 |
| Caprylyl glycol | 1.0 | 0 | 1.0 | 0 | 1.0 | 0 | 1.0 | 1.0 | 1.0 | 1.0 | 1.0 |
| Styrene/Acrylates copolymer | 5.0 | 5.0 | 0 | 0 | 5.0 | 5.0 | 0 | 5.0 | 5.0 | 3.0 | 4.0 |

### Example 1 SPF, UVA/UVB and Critical Wavelength

Sample photoprotective compositions were prepared according to compositions A, B, C, D and E, and were tested for SPF, UVA/UVB ratio and critical wavelength by measuring absorbance on a Labsphere UV-1000S Ultraviolet Transmittance Analyzer. A synthetic skin substrate (VITRO-SKIN substrate by IMS, Inc. of Milford, CN) was used for testing the sunscreen compositions. To prepare the substrate, a 300 gram solution of 44 grams of glycerin and 256 grams of deionized water was added to an IMS hydrating chamber, and a sheet of VITRO-SKIN cut into 6.5 cm squares was placed in the hydrating chamber for approximately 16 hours and was then used for absorbance measurements.

To prepare slides for testing, sunscreen composition was drawn into a pipette and preweighed. An amount of composition was dispensed equal to a dosage rate of 2 mg/cm² was applied to the VITRO SKIN which was located on a foam block covered by parafilm. The test material was spread by finger covered with finger cot, first in a circular motion, then by a side-to-side motion. The square was then mounted in a slide holder and allowed to dry for fifteen minutes before reading. The resulting data and printout are used to report SPF, UVA/UVB ratio and critical wavelength.

The SPF results are summarized in the following Table 1A.

**TABLE 1A**

| Sample formulation | In-vitro SPF |
|---|---|
| A (5 % copolymer spheres, 1 % caprylyl glycol) | 193 |
| B (5 % copolymer spheres, 0% caprylyl glycol) | 163 |
| C (0% copolymer spheres, 1 % caprylyl glycol) | 153 |
| D (0% copolymer spheres, 0% caprylyl glycol) | 134 |
| E (5 % copolymer spheres; 1% caprylyl glycol; no UV filters) | 2 |

Comparing the data in Table 1A for formulations C and D shows that adding 1% caprylyl glycol produces an SPF increase of about 19 units in the absence of styrene/acrylate copolymer spheres. Therefore, one would expect that adding 1% caprylyl glycol to the formulation B, which had an SPF of 163, would provide an SPF of about 182 (=163+19). However, the actual result for such a formulation (formulation A) was SPF 193, clearly illustrating a synergistic effect between the styrene/acrylate copolymer spheres and the glycol that provides an unexpected improved result in the performance of the photoprotective composition.

The U.S. Food and Drug Administration current holds that a raw material in a sunscreen composition must not exhibit an in vivo (human) SPF of 2 or higher. It has been found that, in general, in vitro SPF values determined for photoprotective compositions as described herein are two times greater than the in vivo test results for the same compositions. Therefore, the in vitro SPF measurement of about 2 for formulation E suggests as much as about 10% styrene/acrylate copolymer spheres can be used in sunscreen compositions within U.S. FDA limits. Other countries impose no limit on the amount of styrene/acrylate copolymer spheres than can be incorporated into a sunscreen formulation.

The UVA/UVB ratio of the samples was noted as follows in Table 1B:

**TABLE 1B**

| Sample formulation | UVA/UVB ratio |
|---|---|
| A (5 % copolymer spheres, 1 % caprylyl glycol) | 0.98 |
| B (5 % copolymer spheres, 0% caprylyl glycol) | 0.95 |
| C (0% copolymer spheres, 1 % caprylyl glycol) | 0.95 |
| D (0% copolymer spheres, 0% caprylyl glycol) | 0.93 |
| E (5 % copolymer spheres; 1 % caprylyl glycol; no UV filters) | 0.48 |

The data of Table 1B shows that although the sample according to formulation has very high sunscreen efficiency (i.e., it has a broad spectrum efficacy), it is the combination of styrene/acrylate copolymer spheres and glycol that enhanced the broad spectrum efficacy of a photoprotective composition.

The critical wavelength for each sample was determined, and the results are set forth in the following Table 1C.

**Table 1C**

| Sample formulation | Critical wavelength (nm) |
|---|---|
| A (5 % copolymer spheres, 1% caprylyl glycol) | 384 |
| B (5 % copolymer spheres, 0% caprylyl glycol) | 382 |
| C (0% copolymer spheres, 1 % caprylyl glycol) | 383 |
| D (0% copolymer spheres, 0% caprylyl glycol) | 382 |
| E (5 % copolymer spheres; 1 % caprylyl glycol; no UV filters) | 380 |

The data of Table 1C shows that formulation A exhibits the longest critical wavelength of all of the samples tested, and again demonstrates that enhanced photoprotection is attained by combining styrene/acrylate copolymer spheres with the glycol.

### Example 2 Photostability

Sample photoprotective compositions according to formulations A, F and G were tested for photostability measuring absorbance on a LABSPHERE UV-1000S Ultraviolet Transmittance Analyzer before and after irradiation with a Solar Light Company Model 165 Solar Simulator equipped with a WG 320 filter and a UGI11 filter for continuous emission from 290 nm to 400 nm. Output was monitored by a PMA 2114 UV-A DCS Detector and controlled by a PMA 2100 Automatic Dose Controller (Solar Light Co.)

The test substrate used was roughened PMMA plates. To prepare plates for testing, sunscreen composition was drawn into a pipette and preweighed, and was then dispensed until a dosage of 2 mg/cm² (milligram per square centimeter) was achieved. The test material was spread by finger covered with finger cot, first in a circular motion, then by a side-to-side motion. The square was then mounted in a slide holder and was allowed to dry for 15 minutes before reading.

To test for photostability, the plate was positioned on the UV transmittance analyzer using registration marks, and a scan of a 1 cm² spot on the plate was performed. The plate was then transferred to a holder placed adjacent to the solar simulator and, using calipers, was positioned such that the beam of UV radiation exiting the solar simulator illuminated the same 1 cm² spot on the plate. The following software settings were used: UVB: 290 — 320 nm; UVA: 320 — 400 nm; SPF: 290 — 400 nm; Spectral Irradiance: noon, July 3, Albuquerque, NM; SPF spectral irradiance and erythemal effectiveness settings as set by manufacturer. After an initial exposure of 5 J/cm² (Joules per square centimeter), the plate was again placed in position on the UV transmittance analyzer, and a scan of the exposed spot was performed, followed by an additional 50 J/cm² of irradiation, a scan, and another 50 J/cm² of irradiation, and a scan, all on the same 1 cm² spot. The results are set forth in the following Table 2A.

**TABLE 2A**

| Sample formulation | A (5 % copolymer spheres, 1% caprylyl glycol) | | F (5 % copolymer spheres, 0% caprylyl glycol) | | G (0% copolymer spheres, 1% caprylyl glycol) | |
|---|---|---|---|---|---|---|
| Wavelength (nm) | 310 | 370 | 310 | 370 | 310 | 370 |
| 50 J/cm² | 100 | 100 | 100 | 96.69 | 97.5 | 83.02 |
| 100 J/cm² | 100 | 100 | 100 | 87.61 | 97.67 | 64.49 |

The data in Table 2A clearly demonstrates the improvement of UVB (310 nm) photostability and UVAI (370 nm) photostability in the presence of the combination of 5% styrene/acrylate copolymer spheres and 1% caprylyl glycol. The sample of composition A showed complete photostability in the UVB region (310 nm) and the UVA region (370 nm) after exposure to 50 J/cm² and 100 J/cm² cumulative dose of solar simulator light. The comparative samples exhibited noticeable photodegradation in the UVA1 region.

### Example 3

PFA value is an indicator of a sunscreen's protection in UVA region, which is associated with photoaging, as opposed to the UVB region, which is associated with sunburn. PFA is also related to the degree of photostabilization of UVA sunscreens, such as avobenzone and its derivatives. So a PFA boost to a sunscreen composition will directly correlate with higher UVA efficiency of a sunscreen as well as higher degree of photostabilization of a UVA sunscreen.

A visual end point of Persistent Pigment Darkening (PPD) was used to determine the in-vivo level of protection afforded by a photoprotective composition according to composition A (5% styrene/acrylate copolymer spheres, 1% caprylyl glycol) against UVA radiation. In a 10 subject test conducted using the Japanese Cosmetic Industry Association (JCIA) test method, the photoprotective composition exhibited an average value of 30.48 (95% confidence limits 27.33-33.63) at 180 minutes and qualified to be characterized as a PA+++ UVA protective sunscreen product. Based on the 100% photostability data presented above for the UVAI region (370 nm), the in vivo PFA measurement of 30.48 would be expected to remain constant with no photodegradation. In contrast, the photostability data presented for the comparative samples strongly suggests that this PFA value would decrease, thus reducing product efficacy in the UVA1 region.

### Example 4

Clinical SPF tests of sample photoprotective compositions prepared according to compositions A, H, and I were performed in accordance with the "Sunscreen Drug Products for Over-The-Counter Human Use; Final Monograph" issued by the U.S. FDA, May 21, 1999, Fed. Reg. Vol. 64, No. 98, 27666-27693. The results are set forth in the following Table 4A.

## Claims

1. A photoprotective composition, comprising:
one or more photoactive agents;
caprylyl glycol in an amount of 0.1 wt.% to 10 wt.% based on the total weight of the composition, and
styrene/acrylate copolymer spheres in an amount of 0.1 wt.% to 10 wt.% based on the total weight of the composition.

2. The photoprotective composition of claim 1, wherein the one or more photoactive agents are present in the composition in an amount of 1 wt. % to 40 wt. %, based on the total weight of the composition.

3. The photoprotective composition of claim 1, wherein the photoprotective composition is in a form of an emulsion and further comprises the photoactive agent in an amount of 0.1 wt. % to 40 wt. %, caprylyl glycol in an amount of 0.1 wt. % to 10 wt. %, the styrene/acrylate copolymer spheres in an amount of 0.1 wt. % to 10 wt. %, the water in an amount of 40 wt. % to 90 wt. %, and the emulsifier in an amount of 0.01 wt. % to 10 wt. %, based on the total weight of the composition, and wherein a pH of the composition is from 3 to 9.

4. The photoprotective composition of claims 1 to 3, wherein the one or more photoactive agents is selected from the group consisting of UV-A filters, UV-B filters, pigments, dyes, and any combinations thereof.

5. The photoprotective composition of claims 1 to 4, wherein the one or more photoactive agents is selected from the group consisting of para-aminobenzoic acid, avobenzone, cinoxate, dioxybenzone, homosalate, methyl anthranilate, octocrylene, octyl methoxycinnamate, octyl salicylate, oxybenzone, padimate O, phenylbenzimidazole sulfonic acid, sulisobenzone, titanium dioxide, trolamine salicylate, zinc oxide, diethanolamine methoxycinnamate, ethyl-[bis(hydroxypropyl)]aminobenzoate, glyceryl aminobenzoate, 4-isopropyl dibenzoylmethane, 4-methylbenzylidene camphor, terephthalylidene dicamphor sulfonic acid, sulisobenzone, and any combinations thereof.

6. The photoprotective composition of claims 1 to 5, wherein the caprylyl glycol is present in the composition in an amount of at least one of 0.25 wt. % to 5 wt. %, and 0.50 wt. % to 1.25 wt. %, based on the total weight of the composition.

7. The photoprotective composition of claims 1 to 6, wherein styrene/acrylate copolymer spheres having a mean particle size of 100 micrometers.

8. The photoprotective composition of claim 7, wherein the styrene/acrylate copolymer spheres are hollow.

9. The photoprotective composition of claim 7, wherein the styrene/acrylate copolymer spheres are present in the composition in an amount of at least one of 3 wt. % to 7 wt. %, 4 wt. % to 6 wt. %, 0.25 wt. % to 5 wt- %, and 0.5 wt. % to 1.25 wt. %, based on the total weight of the composition.

10. The photoprotective composition of claims 1 to 9, wherein caprylyl glycol is included in a solvent system to dissolve the one or more photoactive agents.

11. The photoprotective composition of claim 10, wherein the solvent system includes one or more of C₁₂ - 15 alkyl benzoates, capric triglycerides, caprylic triglycerides, diethylhexyl adipate, diethylhexyl malate, diethylhexyl 2,6-naphthalate, ethylhexyl palmitate, ethylhexyl stearate, isoeicosane, isopropyl myristate, isopropyl palmitate, mineral oil, octyldodecyl neopentanoate, polyisobutene, PPG-2 myristyl ether propionate, cocoglycerides, isostearyl linoleate, diisopropyl adipate, myristyl ether myristate, octyl palmitate, propylene glycol ricinoleate, cetyl esters, propylene glycol laurate, and any combinations thereof.

12. The photoprotective composition of claim 3, wherein the emulsifier is comprised of at least one of acrylates crosspolymer, acrylates/C₁₀₋₃₀ alkylacrylate crosspolymer, acrylates/vinyl isodecanoate crosspolymer, polyacrylic acid, sodium polymethacrylate, sodium polyacrylate, polyacrylates, cetyl alcohol, cetearyl alcohol, oleth-10, diethylhexyl esters, sorbitan oleate, sorbitan sesquioleate, sorbitan isostearate, sorbitan trioleate, PEG-20 almond glycerides, polyglyceryl-3-diisostearate, polyglycerol esters of oleic/isostearic acid, polyglyceryl-6 hexaricinolate, polyglyceryl-4-oleate, polygylceryl-4 oleate/PEG-8 propylene glycol cocoate, sodium glyceryl oleate phosphate, hydrogenated vegetable glycerides phosphate, and any combinations thereof.

13. The photoprotective composition of claim 3, wherein the emulsifier is present in an amount of 0.1 wt. % to 5 wt. %, based on the total weight of the composition.

14. The photoprotective composition of claims 1 to 13, further including at least one of a thickening agent, an emollient, a moisturizer, a humectant, a film former/waterproofing agent, a bio-active ingredient, a pH adjuster/chelating agent, a preservative, fragrance, an effect pigment, a color additive, a lubricant, an elastomer, and any combination thereof.

15. The photoprotective composition of claim 14, wherein the film former/waterproofing agent is comprised of at least one of acrylates/acrylamide copolymer, acrylates copolymer, acrylates/C₁₂₋₂₂ alkylmethacrylate copolymer, polyethylene, waxes, VP/Dimethiconylacrylate/Polycarbamyl polyglycol ester, butylated PVP, PVP/Hexadecene copolymer, octadecene/MA copolymer, PVP/Eicosene copolymer, tricontanyl PVP, Brassica Campestris/Aleuritis Fordi Oil copolymer, decamethyl cyclopentasiloxane trimethylsiloxysilicate, and any combinations thereof, and wherein the film former/waterproofing agent is present in an amount of at least one of 0.1 wt. % to 5 wt. %, and 1 wt. % to 3 wt. %, based on the total weight of the composition.
